# NEUE EUROPÄISCHE PATENTSCHRIFT

(11) **EP 0 606 456 B2**
(45) Veröffentlichungstag und Bekanntmachung des Hinweises auf die Entscheidung über den Einspruch: **18.10.2000**
(45) Hinweis auf die Patenterteilung: 20.11.1996
(21) Anmeldenummer: 93917741.6
(22) Anmeldetag: 04.08.1993
(51) Int. Cl.: G01N 27/407, G01N 27/417

(54) **ANORDNUNG ZUM KONTINUIERLICHEN ÜBERWACHEN DER KONZENTRATION VON NO IN GASGEMISCHEN**
ARRANGEMENT FOR CONTINUOUSLY MONITORING THE NO CONCENTRATION IN GAS MIXTURES
DISPOSITIF POUR CONTROLER EN CONTINU LA CONCENTRATION EN NO DANS DES MELANGES GAZEUX

(30) Priorität: 04.08.1992 DE 4225775
(43) Veröffentlichungstag der Anmeldung: 20.07.1994
(73) Patentinhaber: Heraeus Electro-Nite International N.V., 3530 Houthalen (BE)
(72) Erfinder: HÄFELE, Edelbert, D-76228 Karlsruhe (DE); SCHÖNAUER, Ulrich, D-76131 Karlsruhe (DE); GÖPEL, Wolfgang, D-72076 Tübingen (DE); WIEMHÖFER, Hans-Dieter, D-72805 Lichtenstein (DE); REINHARDT, Götz, D-72074 Tübingen (DE)
(74) Vertreter: Kühn, Hans-Christian
(86) Internationale Anmeldenummer: EP9302075
(87) Internationale Veröffentlichungsnummer: WO9403796

(56) Entgegenhaltungen:
- EP-A- 0 006 989
- DE-A- 3 112 739
- DE-A- 3 315 654
- FR-A- 2 358 655
- FR-A- 2 478 817
- US-A- 4 927 517
- Teraoka, T. et al. Chemistry Letters 1988, S. 503-506
- Lehrbuch der Elektrochemie, Gustav Kortüm, 5. Auflage, 1972, Verlag Chemie GmbH Weinheim, Seiten 480-481
- Veröffentlichter Entwurf zur DIN-Norm 50918, Januar 1977, "Korrision der Metalle - Elektrochemische Korrosionsuntersuchungen", Fachausschuss Materialprüfung im Deutschen Institut für Normung e.V., Berlin, Seite 1-5
- Elektrochemical Methods, Fundamentals and Applications, A. Bard und L. Faulkner, 1980, Verlag John Wiley & Sons, Kapitel 13.4 "Potentiostats", Seiten 563-566

## Beschreibung

Die Erfindung betrifft eine Anordnung gemäß dem Oberbegriff des Hauptanspruchs.

Eine solche Vorrichtung ist bekannt. (DE-U-91 03 547). Mit dieser bekannten Vorrichtung ist eine potentiometrische Messung der Konzentration von Stickoxiden, z.B. NO dadurch möglich, daß ohne Stromfluß durch die beiden elektrochemischen Halbzellen die sich von selbst einstellende Potentialdifferenz zwischen einer der Elektroden und der Gegenelektrode gemessen wird.

Der Erfindung liegt demgegenüber die Aufgabe zugrunde, eine gattungsgemäße Anordnung gemäß dem Oberbegriff des Hauptanspruchs so weiter auszugestalten, daß eine Messung von NO mit deutlich verbesserter Empfindlichkeit möglich wird.

Diese Aufgabe wird bei einer Anordnung gemäß dem Oberbegriff des Hauptanspruchs erfindungsgemäß durch dessen kennzeichnende Merkmale gelöst.

Erfindungsgemäß wird hierbei zwischen der Meßelektrode und der Gegenelektrode eine variable Spannungsquelle geschaltet, wodurch sich ein aus der Gegenelektrode austretender elektrischer Strom ergibt, der eine Funktion der Konzentration von NO im Meßgas darstellt. Die angelegte Spannung wird dabei so nachgeführt, daß sich ein konstantes Potential an der Meß- und der Referenzelektrode ergibt. Die gemessene Stromstärke ist durch die Kinetik der ablaufenden Elektrodenreaktionen begrenzt, welche durch die Anwesenheit von NO verändert wird. Deshalb erweist sich als wichtige Grundlage der Auswahl des Elektrodenmaterials die katalytische Wirkung dieses Materials auf NO. Als Elektrodenmaterialien kommen hier perowskitische Verbindungen der Formel Ln₁₋ₓAMO₃ in Frage, wobei hier Ln ein Element der Reihe Sc, Y, La oder ein Vertreter der Lanthaniden (Ordnungszahl 58-71), A zumindest ein Element der zweiten Hauptgruppe, M zumindest ein Element der Übergangsmetalle Cr, Mn, Fe, Co ist und x zwischen 0,01 und 0,5 liegt.

Ferner lassen sich Querempfindlichkeiten, d.h. die Empfindlichkeit auf andere Gase im Gasgemisch durch Wahl des Materials der beiden Elektroden gezielt minimieren.

Zweckmäßige Ausgestaltungen und Weiterbildungen der Erfindung sind in den abhängigen Ansprüchen gekennzeichnet.

Ein bevorzugtes Ausführungsbeispiel wird nachfolgend unter Bezugnahme auf die Zeichnung näher erläutert. In dieser zeigt:

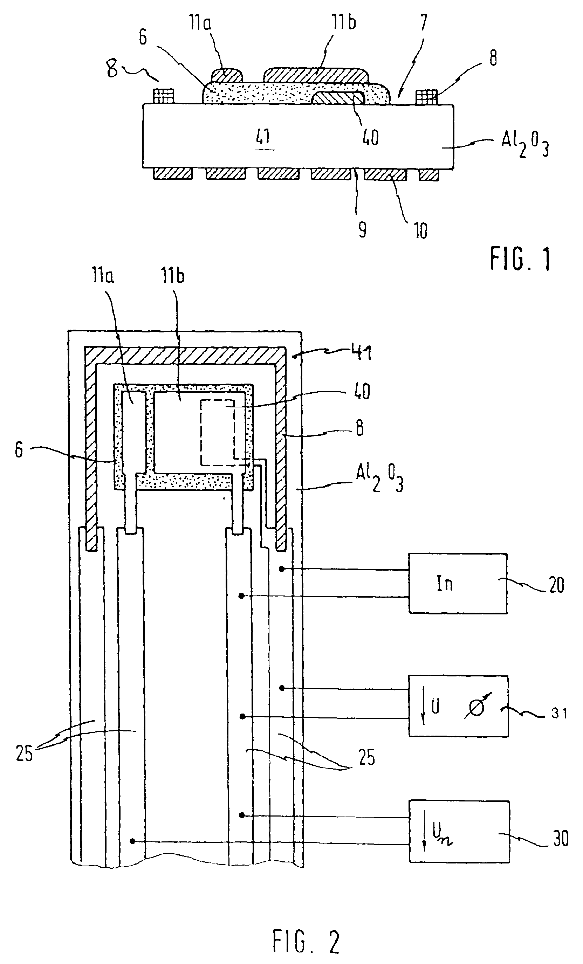

- Figur 1: eine erste Ausführungsform der Anordnung, im schematischen Querschnitt;
- Figur 2: die Anordnung gemäß Fig. 1, in Draufsicht;
- Figur 3: den prinzipiellen Aufbau der Anordnung;
- Figur 4: eine Kennlinienschar des zwischen Meß- und Gegenelektrode fließenden Stromes über der angelegten Spannung U mit der Konzentration an NO als Parameter;
- Figur 5: ein Diagramm des fließenden Stromes in Abhängigkeit von NO; und
- Figur 6: ein Diagramm über einen Langzeittest.

Der Träger 41 für die elektrochemische Zelle, vorzugsweise aus Al₂O₃ weist auf dessen einer Seite 7 die Gegenelektrode 40, darüber eine poröse Schicht eines anionenleitenden Festelektrolyten (6), vorzugsweise yttriumdotiertes Zirkondioxid (8 Mol-%) und darüber die Referenz- 11a und Meßelektrode 11b auf, die nachfolgend noch näher erläutert werden. Bei dieser Anordnung muß die Meßelektrode 11b in Draufsicht die Gegenelektrode 40 gänzlich überdecken. Ferner ist auf der einen Seite 7 des als keramisches Plättchen ausgebildeten Trägers 41 ein die elektrochemische Zelle umschließender Temperaturfühler 8 vorgesehen. Auf der Rückseite 9 des Trägers 41 ist eine Widerstandsheizschicht 10 vorgesehen, die in Draufsicht mäanderförmig aufgebracht sein kann.

Auf der Oberseite der als Festelektrolyt 6 dienenden dünnen Schicht sind zwei verschiedene Elektroden, nämlich eine Referenzelektrode 11a und eine Meßelektrode 11b, die vorzugsweise perowskitische Materialien der Summenformel Ln₁₋ₓAₓMO₃ (x = 0,01 bis 0,5) aufweisen, in Form dünner Schichten aufgebracht, die jeweils voneinander beabstandet angeordnet sind.

Diese mittels Siebdrucktechnik und anschließendem Sintern hergestellte Anordnung in Planar- bzw. Dickschichttechnologie weist eine äußerst geringe Masse und kleine Einbaumaße auf und benötigt daher zur Aufnahme nur ein kleines Gehäuse.

Zum Abgriff des Meßstromes sowie der Zuführung der Meßspannung sowie zur Beheizung der Widerstandsheizschicht 10 sind auf dem Träger 41 Metallschichten in Form von aufgedruckten Leiterbahnen 25 vorgesehen.

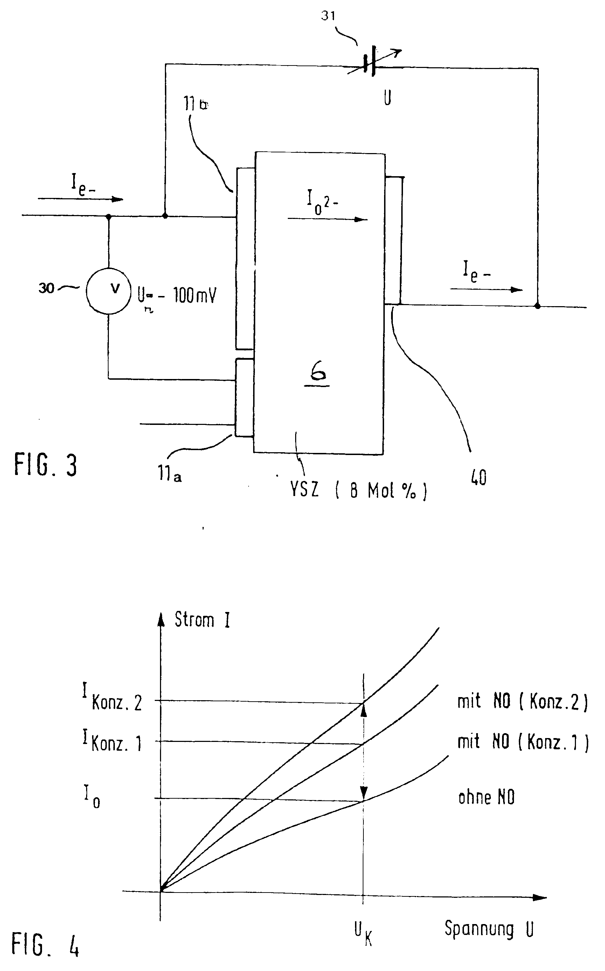

In Fig. 3 ist der prinzipielle Aufbau der eingesetzten amperometrischen Meßanordnung dargestellt. Zwischen der Meßelektrode 11b und der Gegenelektrode 40 wird eine Spannung U angelegt, und der durch die Meßelektrode 11b, den Festelektrolyten 6, vorzugsweise yttriumstabilisiertes Zirkondioxid (YSZ, 8 Mol-%) und die Gegenelektrode 40 fließende Strom I gemessen. Dabei wird die Spannung U so nachgeführt, daß sich eine konstante Potentialdifferenz Uₙ zwischen der Meßelektrode 11b und Referenzelektrode 11a ergibt. In jedem Falle sollte der Festelektrolyt ein Sauerstoffanionenleiter wie beispielsweise ZrO₂ oder CeO₂ sein.

Demgemäß ist in Fig. 2 eine Spannungsquelle 31 zwischen die Zuleitungen 25 der Elektroden 11b und 40, ein Strommeßgerät 20 zwischen der Zuleitung zur Meßelektrode 11b und der Zuleitung zur Gegenelektrode 40 und ein Spannungsmeßgerät 30 zwischen die Zuleitungen der Meß-11b und Referenzelektrode 11a geschaltet. Wie in Fig. 4 gezeigt, ergibt sich für jede Konzentration von NO eine Kurve des Stroms in Abhängigkeit der Spannung. Bei konstanter Spannung U_{K} variiert somit der Strom zwischen dem Wert I₀ und I_{Konz.2}.

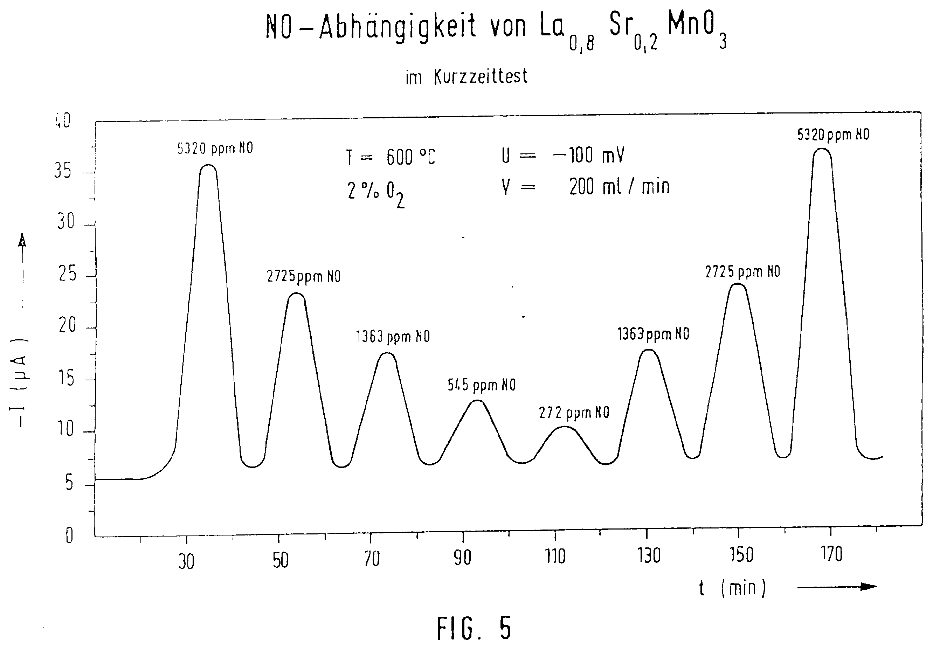

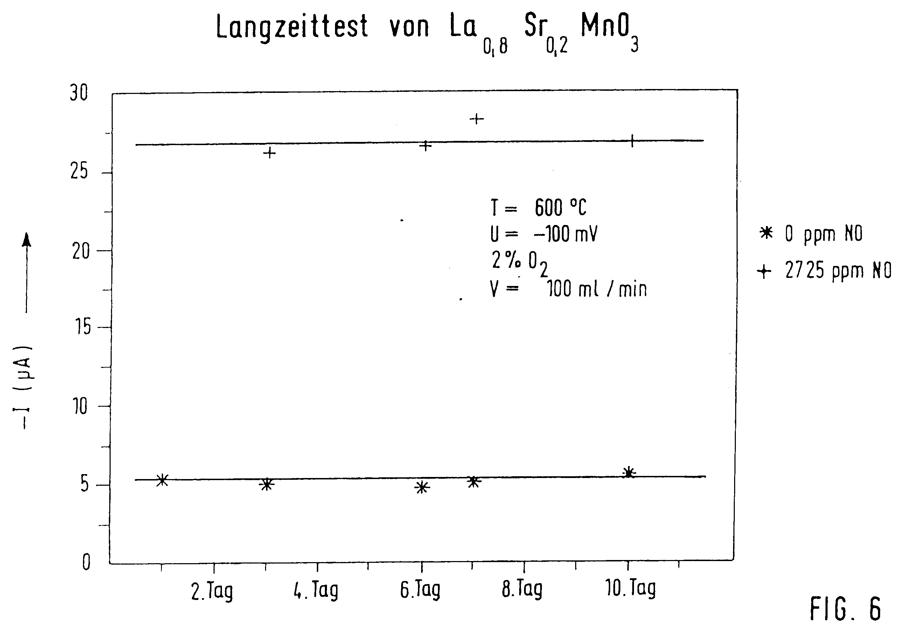

Fig. 5 zeigt in einem Diagramm die Abhängigkeit des Stromes von der Konzentration von NO bei der Messung mit einer erfindungsgemäßen Anordnung mit La_{0,8}Sr_{0,2}MnO₃ als Elektrodenmaterial. Die Langzeitstabilität des Meßsignals (I) bei 0 und 2725 ppm NO-Konzentration ergibt sich ohne weiteres aus dem Diagramm gemäß Fig. 6.

## Patentansprüche

1. Anordnung zur kontinuierlichen Überwachung der Konzentration von Stickstoffoxid, vorzugsweise Stickstoffmonoxid (NO) in Gasgemischen mit einem Träger (41), der zumindest zwei elektrochemische Halbzellen mit einem sauerstoffionenleitenden Festelektrolyten (6) als dünne Schicht und mit einer porösen Festkörperstruktur versehene, vorzugsweise metall- und/oder metalloxidhaltige sowie mit den gasförmigen Bestandteilen wechselwirkende Elektroden (11a, 11b, 40) als dünne Schichten auf einer Seite (7) der beiden Seiten (7, 9) des Trägers (41) aufweist, wobei ein von der Konzentration des NO abhängiges und in Konzentrationswerte umrechenbares elektrisches Meßsignal erzeugt wird, wobei auf der einen, nämlich der Oberseite (7) des Festelektrolyten (6) zumindest zwei der Elektroden als Meßelektrode (11b) und Referenzelektrode (11a) zueinander beabstandet aufgebracht sind und wobei unter dem Festelektrolyten (6) eine Gegenelektrode (40) auf dem Träger (41) angeordnet ist,
**dadurch gekennzeichnet,**
daß die Meßelektrode (11b) perowskitische Verbindungen aufweist, daß zwischen der Meßelektrode (11b) und der Gegenelektrode (40) eine Spannungsquelle (31) geschaltet ist und daß zwischen der Gegenelektrode (40) und der Meßelektrode (11b) ein Strommeßgerät (20) geschaltet ist, mit dem der aus der Gegenelektrode (40) in Abhängigkeit der angelegten Spannung (U) austretende elektrische Strom (I) als Funktion der Konzentration von NO gemessen wird, wobei die angelegte Spannung (U) der Spannungsquelle (31) so nachgeführt wird, daß die Potentialdifferenz (Un) zwischen der Meßelektrode (11b) und der Referenzelektrode (11a) konstant gehalten wird.

2. Anordnung nach Anspruch 1, dadurch gekennzeichnet, daß die Meßelektrode perowskitische Materialien der Formel Ln₁₋ₓAₓMO₃ aufweist, wobei Ln ein Element der Reihe Sc, Y, La oder ein Vertreter der Lanthaniden (Ordnungszahl 58-71), A zumindest ein Element der zweiten Hauptgruppe, M zumindest ein Element der Übergangsmetalle Cr, Mn, Fe, Co ist und x zwischen 0,01 und 0,5 liegt.

3. Anordnung nach Anspruch 1 oder 2, dadurch gekennzeichnet, daß auf der anderen Seite (9) des Trägers (41) eine elektrische Widerstandsheizschicht (10) als Heizung vorgesehen ist.

4. Anordnung nach Anspruch 1 oder 2, dadurch gekennzeichnet, daß auf der einen Seite (7) des Trägers (41) ein von der Schicht des Festelektrolyten (6) und der Meß- (11b) und der Referenzelektrode (11a) beabstandeter Temperaturfühler (8) am Rand des Trägers (41) um die Elektroden (11a, 11b), die dünne Schicht des Festelektrolyten (6) und die Gegenelektrode (40), herumgeführt ist.

5. Anordnung nach einem der Ansprüche 1 bis 4, dadurch gekennzeichnet, daß der Träger (41) als nicht-ionenleitende Keramik ausgebildet ist und auf der einen Seite (7) sowohl die Gegenelektrode (40) als auch den Temperaturfühler (8) sowie den Festelektrolyten (6) als dünne Schicht aufweist, auf dem die Elektroden (11a, 11b) aufgebracht sind.

6. Anordnung nach Anspruch 5, dadurch gekennzeichnet, daß der Träger (41) Aluminiumoxid (Al₂O₃) aufweist.

7. Anordnung nach einem der Ansprüche 4 bis 6, dadurch gekennzeichnet, daß der Träger (41) aus Keramik mit einem Anschlußstück für die elektrischen Zuleitungen (25) versehen ist.

8. Anordnung nach einem der Ansprüche 1 bis 7, dadurch gekennzeichnet, daß der Festelektrolyt (6) ein Anionenleiter ist.

9. Anordnung nach einem der Ansprüche 1 bis 7, dadurch gekennzeichnet, daß der Festelektrolyt (6) stabilisiertes ZrO₂ oder CeO₂ aufweist.

10. Anordnung nach Anspruch 9, dadurch gekennzeichnet, daß der Festelektrolyt (6) yttriumdotiert ist.

11. Anordnung nach Anspruch 10, dadurch gekennzeichnet, daß die Dotierung bevorzugt 8 Mol-% beträgt.

12. Anordnung nach einem der Ansprüche 1 bis 8, dadurch gekennzeichnet, daß alle Schichten, nämlich die der Meß- (11b) und der Referenzelektrode (11a), die Gegenelektrode (40), die dünne Schicht des Festelektrolyten (6), der Temperaturfühler (8) und die Widerstandsheizschicht (10) in Dickschichttechnologie auf dem Träger (41) aufgebracht sind.

13. Anordnung nach Anspruch 12, dadurch gekennzeichnet, daß die Schichten mittels Siebdruck aufgebracht sind.

14. Anordnung nach den Ansprüchen 1 bis 13, dadurch gekennzeichnet, daß die Gegenelektrode (40) und/oder die Referenzelektrode (11a) eine perowskitische Verbindung aufweist.

15. Anordnung nach den Ansprüchen 1 bis 14, dadurch gekennzeichnet, daß die Gegenelektrode (40) und/oder die Referenzelektrode (11a) Ln₁₋ₓAₓMO₃ aufweist, wobei Ln ein Element der Reihe Sc, Y, La oder ein Vertreter der Lanthaniden (Ordnungszahl 58-71) und A zumindest ein Element der 2. Hauptgruppe und M zumindest ein Element der Übergangsmetalle Cr, Mn, Fe oder Co ist.

16. Anordnung nach Anspruch 15, wobei x = 0,01 bis 0,5 ist.

## Claims

1. Arrangement for continuously monitoring the concentration of nitrogen oxide, preferably nitrogen monoxide (NO) in gas mixtures, comprising a carrier (41) which has at least two electrochemical half-cells with an oxygen ion-conducting solid electrolyte (6) as a thin layer and electrodes (11a, 11b, 40) which are provided with a porous solid body structure and which are preferably metal-bearing and/or metal oxide-bearing and which interact with the gaseous constituents, being in the form of thin layers, on one side (7) of the two sides (7, 9) of the carrier, wherein an electrical measurement signal which is dependent on the concentration of the NO and which can be converted into concentration values is produced, wherein at least two of the electrodes are disposed at a spacing from each other as a measuring electrode (11b) and a reference electrode (11a) on the one, namely the top side (7) of the solid electrolyte (6), and wherein a counter-electrode (40) is arranged on the carrier (41) under the solid electrolyte (6), characterised in that the measuring electrode (11b) has perovskitic compounds, that a voltage source (31) is connected between the measuring electrode (11b) and the counter-electrode (40), and that connected between the counter-electrode (40) and the measuring electrode (11b) is a current measuring device (20) for measuring the electric current (I) issuing from the counter-electrode (40) in dependence on the applied voltage (U) as a function of the concentration of NO, wherein the applied voltage (U) of the voltage source (31) is so controlled that the potential difference (Un) between the measuring electrode (11b) and the reference electrode (11a) is kept constant.

2. An arrangement according to claim 1 characterised in that the measuring electrode has perovskitic materials of the formula Ln₁₋ₓAₓMO₃ wherein Ln is an element of the series Sc, Y, La or a representative of the lanthanides (atomic numbers 58-71), A is at least one element from the second main group, M is at least one element from the transition metals Cr, Mn, Fe and Co and x is between 0.01 and 0.5.

3. An arrangement according to claim 1 or 2, characterised in that an electrical resistance heating layer (10) is provided as a heating means on the other side (9) of the carrier (41).

4. An arrangement according to claim 1 or 2, characterised in that on the one side (7) of the carrier (41) a temperature sensor (8) which is spaced from the layer of the solid electrolyte (6) and the measuring electrode (11b) and the reference electrode (11a) is passed at the edge of the carrier (41) around the electrodes (11a, 11b), the thin layer of the solid electrolyte (6) and the counter-electrode (40).

5. An arrangement according to one of claims 1 to 4, characterised in that the carrier (41) is in the form of a non-ion-conducting ceramic and on the one side (7) has both the counter-electrode (40) and also the temperature sensor (8) as well as the solid electrolyte (6), in the form of a thin layer, on which the electrodes (11a, 11b) are disposed.

6. An arrangement according to claim 5, characterised in that the carrier (41) has aluminium oxide (Al₂0₃).

7. An arrangement according to one of claims 4 to 6, characterised in that the carrier (41) of ceramic is provided with a connecting portion for the electrical feed lines (25).

8. An arrangement according to one of claims 1 to 7, characterised in that the solid electrolyte (6) is an anion conductor.

9. An arrangement according to one of claims 1 to 7, characterised in that the solid electrolyte (6) has stabilised ZrO₂ or CeO₂.

10. An arrangement according to claim 9, characterised in that the solid electrolyte (6) is yttrium-doped.

11. An arrangement according to claim 10, characterised in that the doping is preferably 8 molar percent.

12. An arrangement according to one of claims 1 to 8, characterised in that all layers, namely that of the measuring (11b) and the reference (11a) electrodes, the counter-electrode (40), the thin layer of the solid electrolyte (6), the temperature sensor (8) and the resistance heating layer (10) are disposed on the carrier (41) using thick-film technology.

13. An arrangement according to claim 12, characterised in that the layers are applied by means of screen printing.

14. An arrangement according to claims 1 to 13, characterised in that the counter-electrode (40) and/or the reference electrode (11a) has a perovskitic compound.

15. An arrangement according to one of claims 1 to 14, characterised in that the counter-electrode (40) and/or the reference electrode (11a) has Ln₁₋ₓAₓMO₃ wherein Ln is an element in the series Sc, Y, La or a representative of the lanthanides (atomic numbers 58-71) and A is at least one element from the second main group and M is at least one element of the transition metals Cr, Mn, Fe or Co.

16. An arrangement according to claim 15, wherein x = 0.01 to 0.5.

## Revendications

1. Dispositif pour contrôler en continu la concentration en oxyde d'azote, de préférence en monoxyde d'azote (NO), dans des mélanges gazeux, comprenant un support (41) présentant au moins deux demi-cellules électro-chimiques, avec un électrolyte solide (6) conducteur d'ions d'oxygène, sous la forme de couche mince et des électrodes (11a, 11b, 40) munies d'une structure poreuse en corps soudes, de préférence des électrodes contenant un métal et/ou un oxyde de métal et en interaction avec des composants gazeux, sous la forme de couches minces sur l'une (7) des deux faces (7, 9) du support (41), un signal électrique de mesure dont les valeurs sont fonction de la concentration du NO et peuvent être converties en valeurs de concentration étant généré, au moins deux des électrodes étant disposées, comme électrode de mesure (11b) et comme électrode de référence (11a), à un écartement donné l'une par rapport à l'autre sur une face, à savoir la face supérieure (7), de l'électrolyte solide (6), et une contre-électrode (40) étant disposée au-dessous de l'électrolyte solide (6), sur le support (41),
caractérisé en ce que l'électrode de mesure (11b) présente des liaisons pérovskitiques, en ce qu'une source de tension (31) est montée entre l'électrode de mesure (11b) et la contre-électrode (40) et en ce qu'un appareil de mesure de courant (20) est monté entre la contre-électrode (40) et l'électrode de mesure (11b) pour mesurer l'intensité (I) du courant électrique sortant de la contre-électrode (40) suivant la tension (U) appliquée en fonction de la concentration de NO, la tension appliquée (U) de la source de tension (31) étant asservie de telle sorte que la différence de potentiel (Un) entre l'électrode de mesure (11b) et l'électrode de référence (11a) est maintenue à une valeur constante.

2. Dispositif selon la revendication 1, caractérisé en ce que l'électrode de mesure présente des matériaux pérovskitiques de formule Ln₁₋ₓAₓMO₃. Ln étant un élément du groupe Sc, Y, La ou un représentant des lanthanides (nombre atomique de 58 à 71), A étant au moins un élément du deuxième groupe principal, M étant au moins un élément des métaux de transition Cr, Mn, Fe, Co, et x étant compris entre 0,01 et 0,5.

3. Dispositif selon la revendication 1 ou 2, caractérisé en ce qu'une couche chauffante par résistance électrique (10) est prévue comme chauffage sur l'autre face (9) du support (41).

4. Dispositif selon la revendication 1 ou 2, caractérisé en ce qu'un capteur de température (8) est monté sur la face (7) du support (41) et ce, à un écartement donné par rapport à la couche de l'électrolyte solide (6) ainsi qu'à l'électrode de mesure (11b) et l'électrode de référence (11a), sur le bord du support (41) autour des électrodes (11a, 11b), de la couche mince de l'électrolyte solide (6) et de la contre-électrode (40).

5. Dispositif selon l'une quelconque des revendications 1 à 4, caractérisé en ce que le support (41) est réalisé sous la forme d'un élément en céramique non conducteur d'ions et présente, sur la face (7) tant la contre-électrode (40) que le capteur de température (8) ainsi que l'électrolyte solide (6) sous forme d'une couche mince sur lequel sont disposées les électrodes (11a, 11b).

6. Dispositif selon la revendication 5, caractérisé en ce que le support (41) présente de l'oxyde d'aluminium (Al₂O₃).

7. Dispositif selon l'une quelconque des revendications 4 à 6, caractérisé en ce que le support (41) en céramique est muni d'un élément de connexion pour les conducteurs électriques (25).

8. Dispositif selon l'une quelconque des revendications 1 à 7, caractérisé en ce que l'électrolyte solide (6) est un conducteur d'anions.

9. Dispositif selon l'une quelconque des revendications 1 à 7, caractérisé en ce que l'électrolyte solide (6) présente du ZrO₂ ou du CeO₂ stabilisé.

10. Dispositif selon la revendication 9, caractérisé en ce que l'électrolyte solide (6) est dopé d'yttrium.

11. Dispositif selon la revendication 10, caractérisé en ce que le dopage est de préférence de 8 pour cent en moles.

12. Dispositif selon l'une quelconque des revendications 1 à 8, caractérisé en ce que toutes les couches, à savoir celle des électrodes de mesure (11b) et de référence (11a), la contre-électrode (40), la couche mince de l'électrolyte solide (6), le capteur de température (8) et la couche de chauffage par résistance électrique (10) sont appliquées sur le support (41) selon un procédé de la technique des couches épaisses.

13. Dispositif selon la revendication 12, caractérisé en ce que les couches sont appliquées par sérigraphie.

14. Dispositif selon l'une quelconque des revendications 1 à 13, caractérisé en ce que la contre-électrode (40) et/ou l'électrode de référence (11a) présente une liaison pérovskitique.

15. Dispositif selon l'une quelconque des revendications 1 à 14, caractérisé en ce que la contre-électrode (40) et/ou l'électrode de référence (11a) présente du Ln₁₋ₓAₓMO₃, Ln étant un élément du groupe Sc, Y, La ou un représentant des lanthanides (nombre atomique de 58 à 71), A étant au moins un élément du deuxième groupe principal, et M étant au moins un élément des métaux de transition Cr, Mn, Fe ou Co.

16. Dispositif selon la revendication 15, x étant compris entre 0,01 et 0,5.
